# EUROPEAN PATENT SPECIFICATION

(11) **EP 2 863 868 B1**
(45) Date of publication and mention of the grant of the patent: **09.12.2020**
(21) Application number: 13733242.5
(22) Date of filing: 20.06.2013
(51) Int. Cl.: A61K 8/25, A61K 8/37, A61K 8/73, A61Q 19/00, A61K 8/81, A61K 8/891, A61K 8/02, A61K 8/06

(54) **TRANSLUCENT COMPOSITION COMPRISING SILICA AEROGEL PARTICLES**
TRANSLUZENTE ZUSAMMENSETZUNG MIT SILICA-AEROGELPARTIKELN
COMPOSITION TRANSLUCIDE COMPRENANT DES PARTICULES D'AÉROGEL DE SILICE

(30) Priority: 21.06.2012 FR 1255845; 02.07.2012 US 201261667079 P
(43) Date of publication of application: 29.04.2015
(73) Proprietor: L'Oréal, 75008 Paris (FR)
(72) Inventor: MONEUZE, Gaelle, F-75020 Paris (FR); BARRAQUET, Florence, F-94400 Vitry sur Seine (FR)
(74) Representative: L'Oreal
(86) International application number: PCT/EP2013/062860
(87) International publication number: WO 2013/190033

(56) References cited:
- DE-A1- 19 923 648
- DE-A1-102004 039 212
- TODD OSTERGAARD ET AL: "Next-Generation Rheology Control Brings New Formulating Options for Personal Care", 20120101, 1 January 2012 (2012-01-01), pages 1-6, XP007921836,
- Material Safety Data Sheet DOWSIL® VM-2270 Aerogel Fine Particles
- RALF EMMERT: "Quantification of the Soft-Focus Effect", COSMETICS&TOILETRIES, vol. 111, July 1996 (1996-07), pages 57-61,

## Description

The invention relates to a translucent cosmetic composition in the form of an oil-in-water emulsion intended for keratin materials, in particular the skin and the lips, the hair and the nails. The invention also relates to a cosmetic method for treating keratin materials using said composition.

In the cosmetics market there is a great demand for products having appearances that are surprising and different from one another.

It is known how to formulate translucent care products in the form of aqueous or aqueous-alcoholic gels, but these products do not always provide the skin with the desired degree of nutrition and may sometimes leave a sticky residue on the skin.

In order to supply the skin with greater comfort and nutrition, emulsified gels, known as gel-creams, are usually formulated. More generally, these products are in the form of a stable dispersion of fine droplets of oil in an aqueous gel. These products generally contain from a few percent and up to 15% to 20% by weight of fatty phase. They have the advantage of combining great freshness on application and provision of nutrition. These products are of opaque appearance.

Surfactant-free oil-in-water emulsions stabilised by particulates are known from DE 199 23 648 A1.

It is sometimes sought to combine in one and the same product the advantages of the emulsified gel, in particular the obtaining of a freshness on application and a nutritive efficacy, and the translucent appearance of a simple aqueous or aqueous-alcoholic gel.

The applicants have discovered that this requirement could be met by adding silica aerogel particles to a composition of opaque emulsified gel type.

More specifically, one subject of the present invention is a translucent cosmetic composition in the form of an oil-in-water emulsion comprising:
(1) a dispersed fatty phase comprising at least one oil selected from silicone oils and fatty acid esters;
(2) a continuous aqueous phase comprising at least one hydrophilic thickening polymer; and
(3) hydrophobic silica aerogel particles; the ratio of the amount by weight of silica aerogel particles to the amount by weight of oil(s) defined in (1) being greater than 0.06; the composition being free of surfactant(s).

As the composition of the invention is intended for topical application to the skin or integuments, it comprises a physiologically acceptable medium, that is to say a medium compatible with all keratin materials, such as the skin, nails, mucous membranes and keratin fibers (such as the hair or eyelashes).

The composition in accordance with the invention is in the form of an emulsified gel of translucent appearance which makes it possible to obtain good cosmetic properties such as a sensation of freshness on application and a satisfactory nutritive efficacy.

Furthermore, the composition according to the invention makes it possible to obtain compositions that are comfortable and soft on application, having mattifying and soft-focus properties.

Another subject of the present invention is a cosmetic method for making up and/or caring for keratin materials comprising a step of applying a composition as defined above to said materials.

In that which follows, the expression "*at least one*" is equivalent to "one or more" and, unless otherwise indicated, the limits of a range of values are included within this range.

The translucency of the composition is evaluated from the measurement of the transmittance, in the visible range, between 400 and 800 nm, at ambient temperature and atmospheric pressure, using a spectrophotometer. For the purposes of the invention, the composition is considered to be translucent when the value of the transmittance is greater than 25%, the value for which a quarter of the incident light is transmitted through the composition.

For the purposes of the present invention, the term "surfactant" means an amphiphilic molecule, i.e. a molecule that has two parts of different polarity, one being lipophilic (which retains fatty substances) and apolar, and the other hydrophilic (water-miscible) and polar. For the purposes of the present invention, the expression "free of surfactant(s)" means a composition in which the surfactant(s) is (are) present in an amount of active material of less than or equal to 0.1% by weight, preferably less than or equal to 0.05% by weight, more preferably still less than or equal to 0.01% by weight of the total weight of the composition.

### Hydrophobic silica aerogels

Aerogels are ultralight porous materials. The first aerogels were made by Kristler in 1932. They are generally synthesized via a sol-gel process in a liquid medium and then dried, usually by extraction with a supercritical fluid, the one most commonly used being supercritical CO₂. This type of drying makes it possible to avoid shrinkage of the pores and of the material. Other types of drying also make it possible to obtain porous materials starting from gel, namely cryodesiccation, which consists in solidifying the gel at low temperature and in then subliming the solvent, and drying by evaporation. The materials thus obtained are referred to respectively as cryogels and xerogels. The sol-gel process and the various drying operations are described in detail in Brinker C.J. and Scherer G.W., Sol-Gel Science, New York, Academic Press, 1990.

The aerogel particles in accordance with the present invention are hydrophobic aerogel particles.

The expression "hydrophobic aerogel particles" is understood to mean any particle of aerogel type having a water absorption capacity at the wet point of less than 0.1 ml/g, i.e. less than 10 g of water per 100 g of particle.

The wet point corresponds to the amount of water that needs to be added to 1 g of particle in order to obtain a homogeneous paste. This method is derived directly from the method for determining the oil uptake of a powder as described in standard NF T 30-022. The measurements are taken in the same manner by means of the wet point and the flow point, which have, respectively, the following definitions:
wet point: mass expressed in grams per 100 g of product corresponding to the production of a homogeneous paste during the addition of a solvent to a powder.

The wet point is measured according to the following protocol:
Equipment used:
Glass plate (25 x 25 mm)
Spatula (wooden shaft and metal part, 15 x 2.7 mm)
Silk-bristled brush
Balance

The glass plate is placed on the balance and 1 g of aerogel is weighed out. The beaker containing the solvent and the liquid sampling pipette are placed on the balance. The solvent is gradually added to the powder, the whole being regularly blended (every 3 to 4 drops) with the spatula. The mass of solvent required to reach the wet point is noted. The average of three tests will be determined.

The hydrophobic aerogels used according to the present invention are inorganic.

The hydrophobic aerogel particles used in the present invention exhibit a specific surface area per unit of mass (S_{M}) ranging from 200 to 1500 m²/g, preferably from 600 to 1200 m²/g and better still from 600 to 800 m²/g, and a size, expressed as the volume-average diameter (D[0.5]), of less than 1500 µm and preferably ranging from 1 to 30 µm, preferably from 5 to 25 µm, better still from 5 to 20 µm and even better still from 5 to 15 µm.

The specific surface area per unit of mass can be determined by the nitrogen absorption method, known as the BET (Brunauer-Emmett-Teller) method, described in The Journal of the American Chemical Society, Vol. 60, page 309, February 1938 and corresponding to the international standard ISO 5794/1 (appendix D). The BET specific surface area corresponds to the total specific surface area of the particles under consideration.

The sizes of the aerogel particles according to the invention can be measured by static light scattering using a commercial particle size analyzer of MasterSizer 2000 type from Malvern. The data are processed on the basis of the Mie scattering theory. This theory, which is exact for isotropic particles, makes it possible to determine, in the case of non-spherical particles, an "effective" particle diameter. This theory is described in particular in the publication by Van de Hulst, H.C., "Light Scattering by Small Particles", Chapters 9 and 10, Wiley, New York, 1957.

According to an advantageous embodiment, the hydrophobic aerogel particles used in the present invention have a specific surface area per unit of mass (S_{M}) ranging from 600 to 800 m²/g and a size, expressed as the volume-average diameter (D[0.5]), ranging from 5 to 20 µm and better still from 5 to 15 µm.

The hydrophobic aerogel particles used in the present invention may advantageously have a tapped density ρ ranging from 0.02 g/cm³ to 0.10 g/cm³ and preferably from 0.02 g/cm³ to 0.08 g/cm³.

In the context of the present invention, this density may be assessed according to the following protocol, known as the tapped density protocol:
40 g of powder are poured into a measuring cylinder; the measuring cylinder is then placed on the Stav 2003 machine from Stampf Volumeter; the measuring cylinder is subsequently subjected to a series of 2500 tapping actions (this operation is repeated until the difference in volume between 2 consecutive tests is less than 2 %); and then the final volume Vf of tapped powder is measured directly on the measuring cylinder. The tapped density is determined by the ratio m/Vf, in this instance 40/Vf (Vf being expressed in cm³ and m in g).

According to one embodiment, the hydrophobic aerogel particles used in the present invention have a specific surface area per unit of volume S_{V} ranging from 5 to 60 m²/cm³, preferably from 10 to 50 m²/cm³ and better still from 15 to 40 m²/cm³.

The specific surface area per unit of volume is given by the relationship: S_{V} = S_{M}.ρ where ρ is the tapped density expressed in g/cm³ and S_{M} is the specific surface area per unit of mass expressed in m²/g, as defined above.

Preferably, the hydrophobic aerogel particles according to the invention have an oil absorption capacity, measured at the wet point, ranging from 5 to 18 ml/g, preferably from 6 to 15 ml/g and better still from 8 to 12 ml/g of particles.

The absorption capacity measured at the wet point, denoted Wp, corresponds to the amount of oil which needs to be added to 100 g of particles in order to obtain a homogeneous paste. It is measured according to the "wet point" method or method of determination of oil uptake of a powder described in standard NF T 30-022. It corresponds to the amount of oil adsorbed onto the available surface of the powder and/or absorbed by the powder by measurement of the wet point, described below:
An amount m = 2 g of powder is placed on a glass plate and the oil (isononyl isononanoate) is then added dropwise. After addition of 4 to 5 drops of oil to the powder, mixing is performed using a spatula, and addition of oil is continued until conglomerates of oil and powder have formed. From this point, the oil is added at the rate of one drop at a time and the mixture is subsequently triturated with the spatula. The addition of oil is stopped when a firm and smooth paste is obtained. This paste must be able to be spread over the glass plate without cracks or the formation of lumps. The volume Vs (expressed in ml) of oil used is then noted.

The oil uptake corresponds to the ratio Vs/m. The aerogel particles used are inorganic and are more particularly hydrophobic silica aerogel particles having the properties stated previously. Silica aerogels are porous materials obtained by replacing (especially by drying) the liquid component of a silica gel with air.

They are generally synthesized via a sol-gel process in a liquid medium and then dried, usually by extraction with a supercritical fluid, the one most commonly used being supercritical CO₂. This type of drying makes it possible to avoid shrinkage of the pores and of the material. The sol-gel process and the various drying operations are described in detail in Brinker C.J. and Scherer G.W., Sol-Gel Science, New York, Academic Press, 1990.

The hydrophobic silica aerogels used according to the present invention are preferably silylated silica (INCI name: silica silylate) aerogels.

The expression "hydrophobic silica" is understood to mean any silica whose surface is treated with silylating agents, for example halogenated silanes such as alkylchlorosilanes, siloxanes, in particular dimethylsiloxanes such as hexamethyldisiloxane, or silazanes, so as to functionalize the OH groups with Si-Rn silyl groups, for example trimethylsilyl groups.

As regards the preparation of hydrophobic silica aerogel particles that have been surface-modified by silylation, reference may be made to document US 7 470 725.

Use will in particular be made of aerogel particles of hydrophobic silica surface-modified with trimethylsilyl groups.

Mention may be made, as hydrophobic silica aerogels which can be used in the invention, for example, of the aerogel sold under the name VM-2260 (INCI name: Silica silylate) by Dow Corning, the particles of which have a mean size of approximately 1000 microns and a specific surface area per unit of mass ranging from 600 to 800 m²/g.

Mention may also be made of the aerogels sold by the company Cabot under the references Aerogel TLD 201, Aerogel OGD 201 and Aerogel TLD 203, Enova® Aerogel MT 1100 and Enova Aerogel MT 1200.

Use will more particularly be made of the aerogel sold under the name VM-2270 (INCI name: Silica silylate) by the company Dow Corning, the particles of which have a mean size ranging from 5 to 15 microns and a specific surface area per unit of mass ranging from 600 to 800 m²/g.

Use will also be made of the aerogel sold under the name Enova® Aerogel MT 1100 (INCI name: Silica silylate) by the company Cabot, the particles of which have a mean size ranging from 2 to 25 microns and a specific surface area per unit of mass ranging from 600 to 800 m²/g.

The hydrophobic silica aerogel particles may be present in the composition according to the invention in a content of active materials ranging from 0.001% to 10% by weight, preferably from 0.005% to 5% by weight, and better still from 0.1% to 2% by weight relative to the total weight of the composition.

### Hydrophilic thickening polymers

The expressions "thickening polymer" or "gelling polymer" are understood, for the purposes of the present invention, to mean a polymer which makes it possible to significantly increase the viscosity of the composition.

The expression "hydrophilic thickening polymer" is understood, for the purposes of the present invention, to mean a water-dispersible thickening polymer.

The hydrophilic thickening polymer(s) used within the context of the present invention is (are) not amphiphilic. They are devoid of a part that is lipophilic (which retains fatty substances) and apolar, in particular devoid of fatty chains.

The hydrophilic thickening polymer(s) may in particular be selected from:
1) acrylic acid homopolymers or copolymers, which are preferably crosslinked, and salts thereof such as the products sold under the names Carbopol® (INCI name: carbomer) by the company Lubrizol; mention may in particular be made of the commercial references Carbopol® 910, 934, 940, 941, 934 P and 981. Mention may also be made of Synthalen K® sold by the company 3V.
2) polyacrylate salts.
   These are in particular neutralized, crosslinked or uncrosslinked polymers in particulate or non-particulate form.

Advantageously sodium salts are preferred.

Mention may for example be made of the polymers bearing the INCI name sodium polyacrylate, such as for example:
- Cosmedia SP® or crosslinked sodium polyacrylate containing 90% solids and 10% water, Cosmedia SPL® or sodium polyacrylate as an inverse emulsion containing about 60% dry active material, an oil (hydrogenated polydecene) and a surfactant (PPG-5 Laureth-5), both sold by the company Cognis,
- partially neutralized crosslinked sodium polyacrylates that are in the form of an inverse emulsion comprising at least one polar oil, for example the product sold under the name Luvigel® EM sold by the company BASF,
   3) 2-acrylamido-2-methylpropanesulfonic acid (AMPS®) polymers and copolymers, which are optionally crosslinked and/or neutralized, selected from:
   a) poly(2-acrylamido-2-methylpropanesulfonic acid) polymers, in particular homopolymers, polyAMPS®, which are crosslinked and neutralized to at least 90%.

They are generally characterized by the fact that they comprise, randomly distributed:
i) from 90% to 99.9% by weight of units of general formula (1) below: 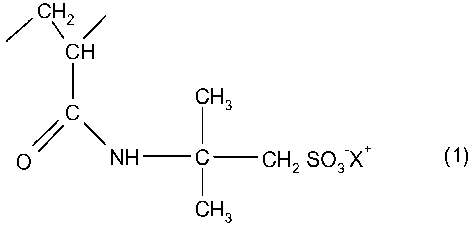 in which X⁺ denotes a cation or a mixture of cations, it being possible for at least 10 mol% of the X⁺ cations to be protons H⁺; and
ii) from 0.01% to 10% by weight of crosslinking units originating from at least one monomer containing at least two olefinic double bonds; the weight proportions being defined relative to the total weight of the polymer.

This polymer preferably comprises from 98% to 99.5% by weight of units of formula (1) and from 0.2% to 2% by weight of crosslinking units.

The X⁺ cation represents a cation or a mixture of cations selected in particular from a proton, an alkali metal cation, a cation equivalent to that of an alkaline-earth metal, or an ammonium ion. The preferred X⁺ cation is the NH₄⁺ cation. More particularly, 90 to 100 mol% of the cations are NH₄⁺ cations and 0 to 10 mol% are protons (H⁺).

The crosslinking monomers containing at least two olefinic double bonds are selected, for example, from dipropylene glycol diallyl ether, polyglycol diallyl ethers, triethylene glycol divinyl ether, hydroquinone diallyl ether, tetraallyloxyethane or other polyfunctional alcohol allyl or vinyl ethers, tetraethylene glycol diacrylate, triallylamine, trimethylolpropane diallyl ether, methylenebisacrylamide or divinylbenzene.

The crosslinking monomers containing at least two olefinic double bonds are more particularly selected from those corresponding to the general formula (2) below: 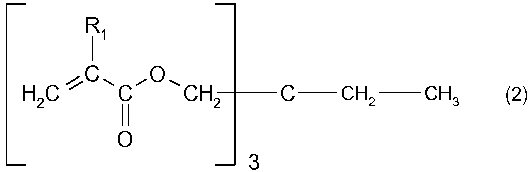 in which R₁ denotes a hydrogen atom or a C₁-C₄ alkyl radical and more particularly the methyl radical (trimethylolpropane triacrylate).

A polymer of this type that may especially be mentioned is the crosslinked and neutralized 2-acrylamido-2-methylpropanesulfonic acid homopolymer sold by the company Clariant under the trade name Hostacerin® AMPS (CTFA name: ammonium polyacryldimethyltauramide). b) copolymers of acrylamide and of AMPS®, in particular crosslinked anionic copolymers of acrylamide and of AMPS®.

These copolymers may especially be crosslinked with a polyolefinically unsaturated compound such as those selected from the group consisting of tetraallyloxyethane, allyl pentaerythritol, methylenebisacrylamide, allyl sucrose and pentaerythritol. Use is preferably made of methylenebisacrylamide, partially or totally neutralized with a neutralizing agent such as sodium hydroxide, potassium hydroxide, aqueous ammonia or an amine such as triethanolamine.

Preferably, said polyolefinically unsaturated compound is present in the copolymer in a concentration of between 0.06 and 1 mmol per mole of the mixture of monomers.

The preferred copolymers are obtained by radical copolymerization of 15-85 mol% of acrylamide and of 15-85 mol% of 2-acrylamido-2-methylpropanesulfonic acid, in particular of 30-70 mol% of acrylamide and of 30-70 mol% of 2-acrylamido-2-methylpropanesulfonic acid, and better still of 55-70 mol% of acrylamide and of 30-45 mol% of 2-acrylamido-2-methylpropanesulfonic acid.

In addition, 2-acrylamido-2-methylpropanesulfonic acid may generally be at least partially neutralized in the form of a salt, for example with sodium hydroxide, with potassium hydroxide or with a low molecular weight amine such as triethanolamine, or mixtures thereof. The gelling agent capable of being used may also be selected from crosslinked anionic copolymers of acrylamide and of 2-acrylamido-2-methylpropanesulfonic acid.

A crosslinked copolymer that is particularly preferred within the context of the implementation of the present invention is especially available under the name Sepigel® 305 sold by SEPPIC (CTFA name: polyacrylamide/C13-14 isoparaffin/Laureth 7); mention may also be made of the product Simulgel® 600 (CTFA name: acrylamide/sodium acryloyldimethyltaurate copolymer/isohexadecane/polysorbate 80) sold by SEPPIC.

### (4) Polysaccharides

Among the polysaccharides, mention may be made of heterogeneous polysaccharides.

The expression "heterogeneous polysaccharide" is understood according to the present invention to mean polymers consisting of the combination of different monosaccharides or of monosaccharides having the same empirical chemical formula but with a different geometric configuration (for example D and L isomers).

These polymers defer both from polyheterosides, which consist of one or more monosaccharides and of a non-carbohydrate part, and homogeneous polysaccharides, which result from the combination of the same monosaccharide.

Thus, the heterogeneous polysaccharide according to the invention consists solely of monosaccharides and results from the combination of at least two different monosaccharides.

The polysaccharides according to the invention may consist of 2 to 10 monosaccharides, compounds commonly referred to as oligosaccharides, or of more than 10 monosaccharides, compounds commonly referred to as polysaccharides.

The monosaccharides present in the polysaccharide according to the invention may be selected from any monosaccharide of natural or synthetic origin that can be envisaged, and in particular such as:
- aldoses, for instance
   - pentoses: ribose, arabinose, xylose or apiose, for example,
   - hexoses: glucose, fucose, mannose or galactose, for example,
- ketoses such as fructose,
- deoxymonosaccharides, such as rhamnose, digitoxose, cymarose or oleandrose,
- monosaccharide derivatives such as uronic acids, for instance mannuronic acid, guluronic acid, galacturonic acid or glycuronic acid, or else itols, for instance mannitol or sorbitol.

The polysaccharide according to the invention may be branched or linear. It may also be substituted, for example with fatty chains, especially comprising 8 to 30 carbon atoms. Moreover, the polysaccharide according to the invention may be an alginate (polymannuronate and polyguluronate) such as a sodium alginate, a propylene glycol alginate, a calcium alginate or a glyceryl alginate.

However, the heterogeneous polysaccharide preferably comprises at least one fucose unit, which may be present in an amount of 10-90% by weight and preferably 15-35% by weight relative to the weight of polysaccharide solids.

In particular, the polysaccharide according to the invention may comprise fucose, galactose and galacturonic acid units, and, for example, may comprise a linear sequence of α-L-fucose, α-D-galactose and galacturonic acid. In this case, it preferably has a viscosity of 800-1200 mPa.s (Brookfield LV31 viscosity, 12 rpm, at 30°C) when it is put into solution in water at a concentration of about 1% by weight. Such a polysaccharide is especially available in the form of a solution in water from the company Solabia under the trade name Fucogel 1000 PP® or Fucogel 1.5P®.

Among the polysaccharides, mention may also be made of the polysaccharides such as:
- algal extracts, such as alginates, carrageenans and agar-agar, and mixtures thereof. Examples of carrageenans that may be mentioned include Satiagum UTC30® and UTC10® from the company Degussa; an alginate that may be mentioned is the sodium alginate sold under the name Kelcosol® by the company ISP;
- gums, such as xanthan gum, guar gum and nonionic derivatives thereof (hydroxypropyl guar), gum arabic, konjac gum or mannan gum, gum tragacanth, ghatti gum, karaya gum, locust bean gum; examples that may be mentioned include the guar gum sold under the name Jaguar HP 105® by the company Rhodia; mannan gum and konjac gum® (1% glucomannan) sold by the company GfN;
- modified or unmodified starches, such as those obtained, for example, from cereals, for instance wheat, maize or rice, from legumes, for instance yellow pea, from tubers, for instance potatoes or cassava, and tapioca starches; dextrins, such as maize dextrins; examples that may especially be mentioned include the rice starch Remy DR I® sold by the company Remy; the maize starch B® from the company Roquette; the potato starch modified with 2-chloroethylaminodipropionic acid neutralized with sodium hydroxide, sold under the name Structure Solanace® by the company National Starch; the native tapioca starch powder sold under the name Tapioca pure® by the company National Starch;
- dextrins, such as dextrin extracted from maize under the name Index® from the company National Starch;
- celluloses and derivatives thereof, in particular alkyl or hydroxyalkyl celluloses; mention may especially be made of methyl celluloses, hydroxyalkyl celluloses, ethyl hydroxyethyl celluloses and carboxymethyl celluloses. Examples that may be mentioned include the cetyl hydroxyethyl celluloses sold under the names Polysurf 67CS® and Natrosol Plus 330® from Aqualon;
- and mixtures thereof.

### 5) Glyceryl acrylate polymers

These glyceryl acrylate polymers are especially selected from copolymers of glyceryl acrylate and of acrylic acid. Such copolymers are especially sold under the names Lubrajel® MS, Lubrajel® CG, Lubrajel® DV, Lubrajel® NP, Lubrajel® Oil, Lubrajel® Oil BG, Lubrajel® PF, Lubrajel® TW, Lubrajel® WA by the company Guardian Laboratories. Use is preferably made of Lubrajel® MS.

The composition according to the invention may comprise a mixture of the various thickening polymers mentioned above.

According to one particular embodiment, the composition according to the invention comprises at least one hydrophilic thickening polymer selected from:
1) acrylic acid homopolymers or copolymers, which are preferably crosslinked, and salts thereof;
2) polyacrylate salts;
3) 2-acrylamido-2-methylpropanesulfonic acid (AMPS®) polymers and copolymers, which are optionally crosslinked and/or neutralized, selected from:
   a) poly(2-acrylamido-2-methylpropanesulfonic acid) polymers, in particular homopolymers, polyAMPS®, which are crosslinked and neutralized to at least 90 %;
   b) copolymers of acrylamide and of AMPS®, in particular crosslinked anionic copolymers of acrylamide and of AMPS®;
4) polysaccharides such as gums, in particular hydroxypropyl guar.

According to one preferred embodiment, the composition according to the invention comprises at least one hydrophilic thickening polymer selected from acrylic acid homopolymers or copolymers, which are preferably crosslinked, and salts thereof; 2-acrylamido-2-methylpropanesulfonic acid (AMPS®) polymers and copolymers, which are optionally crosslinked and/or neutralized, selected from poly(2-acrylamido-2-methylpropanesulfonic acid) polymers, in particular homopolymers, polyAMPS®, which are crosslinked and neutralized to at least 90%, and copolymers of acrylamide and of AMPS®, in particular crosslinked anionic copolymers of acrylamide and of AMPS®.

The hydrophilic thickening polymer(s) may be present in the composition according to the invention in an amount of active materials of between 0.01% and 20% by weight, preferably between 0.05% and 10% by weight of the total weight of the composition.

### Fatty phase

The composition in accordance with the invention comprises at least one dispersed fatty phase.

According to one particular embodiment, the proportion of the fatty phase of the composition in accordance with the invention is less than or equal to 25% by weight, preferably from 0.1% to 20% by weight and better still from 1% to 20% by weight relative to the total weight of the composition.

The nature of the fatty phase (or oily phase) of the emulsion is not critical. The fatty phase may thus consist of any non-waxy fatty substance conventionally used in the cosmetic or dermatological fields; it especially comprises a least one oil, fatty substance that is liquid at 25°C.

The oil or oils are selected from silicone oils and fatty acid esters as described below.

Mention may be made, as oils which can be used in the composition of the invention, for example, of:
- hydrocarbon-based oils of plant origin, such as liquid triglycerides of fatty acids comprising from 4 to 10 carbon atoms, such as heptanoic or octanoic acid triglycerides, or alternatively, for example, sunflower oil, maize oil, soybean oil, marrow oil, grapeseed oil, sesame seed oil, hazelnut oil, apricot oil, macadamia oil, arara oil, castor oil, avocado oil, caprylic/capric acid triglycerides, such as those sold by Stearineries Dubois or those sold under the names Miglyol 810, 812 and 818 by the company Dynamit Nobel, jojoba oil and shea butter oil;
- synthetic esters and ethers, in particular of fatty acids, such as oils of formulae R^{a}COOR^{b} and R^{a}OR^{b} in which R^{a} represents the residue of a fatty acid comprising from 8 to 29 carbon atoms and R^{b} represents a branched or unbranched hydrocarbon-based chain containing from 3 to 30 carbon atoms, such as, for example, purcellin oil, isononyl isononanoate, isopropyl myristate, 2-ethylhexyl palmitate, 2-octyldodecyl stearate, 2-octyldodecyl erucate or isostearyl isostearate; hydroxylated esters, such as isostearyl lactate, octyl hydroxystearate, octyldodecyl hydroxystearate, diisostearyl malate, triisocetyl citrate, fatty alcohol heptanoates, octanoates and decanoates; polyol esters, such as propylene glycol dioctanoate, neopentyl glycol diheptanoate and diethylene glycol diisononanoate; and pentaerythritol esters, such as pentaerythrityl tetraisostearate;
- silicone oils, for instance volatile or nonvolatile polymethylsiloxanes (PDMSs) with a substantially linear or cyclic silicone chain, which are liquid or pasty at ambient temperature, especially cyclopolydimethylsiloxanes (cyclomethicones) such as cyclohexadimethylsiloxane and cyclopentadimethylsiloxane; polydimethylsiloxanes comprising alkyl, alkoxy or phenyl groups, which are pendent or at the end of a silicone chain, these groups containing from 2 to 24 carbon atoms; phenylsilicones, for instance phenyl trimethicones, phenyl dimethicones, phenyltrimethylsiloxydiphenylsiloxanes, diphenyl dimethicones, diphenylmethyldiphenyl-trisiloxanes or 2-phenylethyl trimethylsiloxy silicates, and polymethylphenylsiloxanes;
- mixtures thereof.

In the list of the abovementioned oils, the term "hydrocarbon-based oil" is understood to mean any oil predominantly comprising carbon and hydrogen atoms, and optionally ester, ether, fluoro, carboxylic acid and/or alcohol groups.

The other fatty substances that may be present in the fatty phase are for example selected from pasty fatty substances.

For the purposes of the present invention, the term "pasty fatty substance" is intended to mean a lipophilic fatty compound that undergoes a reversible solid/liquid change in state, which exhibits, in the solid state, an anisotropic crystalline arrangement and which comprises, at a temperature of 23°C, a liquid fraction and a solid fraction.

In other words, the initial melting point of the pasty fatty substance may be less than 23°C. The liquid fraction of the pasty fatty substance, measured at 23°C, may represent from 9% to 97% by weight of the pasty fatty substance. This liquid fraction at 23°C preferably represents between 15% and 85% and more preferably between 40% and 85% by weight.

For the purposes of the invention, the melting point corresponds to the temperature of the most endothermic peak observed in thermal analysis (DSC) as described in the standard ISO 11357-3; 1999. The melting point of a pasty fatty substance can be measured using a differential scanning calorimeter (DSC), for example the calorimeter sold under the name MDSC 2920 by TA Instruments.

The measurement protocol is as follows:
A sample of 5 mg of pasty fatty substance placed in a crucible is subjected to a first temperature rise ranging from -20°C to 100°C, at a heating rate of 10°C/minute, is then cooled from 100°C to -20°C at a cooling rate of 10°C/minute and is finally subjected to a second temperature rise ranging from -20°C to 100°C, at a heating rate of 5°C/minute. During the second temperature rise, the variation in the difference in power absorbed by the empty crucible and by the crucible containing the sample of pasty fatty substance is measured as a function of the temperature. The melting point of the pasty fatty substance is the value of the temperature corresponding to the top of the peak of the curve representing the variation in the difference in power absorbed as a function of the temperature.

The liquid fraction by weight of the pasty fatty substance at 23°C is equal to the ratio of the enthalpy of fusion consumed at 23°C to the enthalpy of fusion of the pasty fatty substance.

The enthalpy of fusion of the pasty fatty substance is the enthalpy consumed by the latter in order to pass from the solid state to the liquid state. The pasty fatty substance is said to be in the solid state when all of its mass is in crystalline solid form. The pasty fatty substance is said to be in the liquid state when all of its mass is in liquid form.

The enthalpy of fusion of the pasty fatty substance is equal to the area under the curve of the thermogram obtained using a differential scanning calorimeter (DSC), such as the calorimeter sold under the name MDSC 2920 by the company TA Instruments, with a temperature rise of 5°C or 10°C per minute, according to the standard ISO 11357-3:1999. The enthalpy of fusion of the pasty fatty substance is the amount of energy required to make the pasty fatty substance change from the solid state to the liquid state. It is expressed in J/g. The enthalpy of fusion consumed at 23 °C is the amount of energy absorbed by the sample to change from the solid state to the state that it exhibits at 23°C, consisting of a liquid fraction and a solid fraction.

The liquid fraction of the pasty fatty substance measured at 32°C preferably represents from 30% to 100% by weight of the pasty fatty substance, preferably from 50% to 100%, more preferably from 60% to 100% by weight of the pasty fatty substance. When the liquid fraction of the pasty fatty substance measured at 32°C is equal to 100%, the temperature of the end of the melting range of the pasty fatty substance is less than or equal to 32°C.

The liquid fraction of the pasty fatty substance measured at 32°C is equal to the ratio of the enthalpy of fusion consumed at 32°C to the enthalpy of fusion of the pasty fatty substance. The enthalpy of fusion consumed at 32°C is calculated in the same way as the enthalpy of fusion consumed at 23°C.

The pasty fatty substance is preferably selected from synthetic fatty substances and fatty substances of plant origin. A pasty fatty substance may be obtained by synthesis from starting materials of plant origin.

The pasty fatty substance is advantageously selected from:
- lanolin and its derivatives,
- polyol ethers selected from pentaerythrityl ethers of a polyalkylene glycol, fatty alcohol ethers of a sugar, and mixtures thereof, the pentaerythrityl ether of polyethylene glycol comprising 5 oxyethylene units (5 OE) (CTFA name: PEG-5 Pentaerythrityl Ether), the pentaerythrityl ether of polypropylene glycol comprising 5 oxypropylene (5 OP) units (CTFA name: PPG-5 Pentaerythrityl Ether), and mixtures thereof, and more especially the PEG-5 Pentaerythrityl Ether, PPG-5 Pentaerythrityl Ether and soybean oil mixture, sold under the name Lanolide by the company Vevy, in which mixture the constituents are in a 46/46/8 ratio by weight: 46% PEG-5 Pentaerythrityl Ether, 46% PPG-5 Pentaerythrityl Ether and 8% soybean oil,

- polymeric or nonpolymeric silicone compounds,
- polymeric or nonpolymeric fluorinated compounds,
- vinyl polymers, in particular:
   ▪ olefin homopolymers and copolymers,
   ▪ hydrogenated diene homopolymers and copolymers,
- liposoluble polyethers resulting from polyetherification between one or more C₂-C₁₀₀ and preferably C₂-C₅₀ diols,
- esters,
- mixtures of hydrocarbons of petrochemical origin, such as for example petroleum jelly paste,
- and/or mixtures thereof.

The pasty fatty substance is preferably a polymer, in particular a hydrocarbon-based polymer.

Preference is given in particular, among the liposoluble polyethers, to copolymers of ethylene oxide and/or of propylene oxide with long-chain C₆-C₃₀ alkylene oxides, more preferably such that the ratio by weight of the ethylene oxide and/or of the propylene oxide to the alkylene oxides in the copolymer is from 5:95 to 70:30. In this family, mention will in particular be made of copolymers such as long-chain alkylene oxides arranged in blocks having an average molecular weight of from 1000 to 10 000, for example a polyoxyethylene/polydodecyl glycol block copolymer, such as the ethers of dodecanediol (22 mol) and of polyethylene glycol (45 OE) sold under the brand name Elfacos ST9 by Akzo Nobel.

Preference is given in particular, among the esters, to:
- esters of a glycerol oligomer, in particular diglycerol esters, especially condensates of adipic acid and of glycerol, for which a portion of the hydroxyl groups of the glycerols has reacted with a mixture of fatty acids, such as stearic acid, capric acid, isostearic acid and 12-hydroxystearic acid, such as in particular those sold under the brand name Softisan 649 by Sasol,
- arachidyl propionate, sold under the brand name Waxenol 801 by Alzo,
- phytosterol esters,
- fatty acid triglycerides and derivatives thereof,
- pentaerythritol esters,
- esters of a diol dimer and of a diacid dimer, where appropriate esterified on their free alcohol or acid functional group(s) by acid or alcohol radicals, in particular dimer dilinoleate esters; such esters can be chosen in particular from esters with the following INCI nomenclature: bis-behenyl/isostearyl/phytosteryl dimer dilinoleyl dimer dilinoleate (Plandool G), phytosteryl isostearyl dimer dilinoleate (Lusplan PI-DA or Lusplan PHY/IS-DA), phytosteryl/isostearyl/cetyl/stearyl/behenyl dimer dilinoleate (Plandool H or Plandool S), and mixtures thereof,
- mango butter, such as the product sold under the reference Lipex 203 by the company AarhusKarlshamn,
- hydrogenated soybean oil, hydrogenated coconut oil, hydrogenated rapeseed oil or mixtures of hydrogenated vegetable oils, such as the soybean, coconut, palm and rapeseed hydrogenated vegetable oil mixture, for example the mixture sold under the reference Akogel® by the company AarhusKarlshamn (INCI name: Hydrogenated Vegetable Oil),
- shea butter, in particular the product having the INCI name Butyrospermum Parkii Butter, such as that sold under the reference Sheasoft® by the company AarhusKarlshamn,
- cocoa butter, in particular the product which is sold under the name CT Cocoa Butter Deodorized by the company Dutch Cocoa BV or the product which is sold under the name Beurre De Cacao NCB HD703 758 by the company Barry Callebaut,
- shorea butter, in particular the product which is sold under the name Dub Shorea T by the company Stearinerie Dubois,
- and mixtures thereof.

According to one preferred embodiment, the pasty fatty substance(s) is (are) selected from petroleum jelly paste, shea butter and cocoa butter.

These fatty substances may be selected in a varied manner by those skilled in the art so as to prepare a composition having the desired properties, for example of translucency.

### Aqueous phase

The aqueous phase of the composition according to the invention comprises at least water. According to the formulation form of the composition, the amount of aqueous phase can range from 75% to 99.9 % by weight, preferably from 80% to 99.9 % by weight, better still from 80% to 98% by weight and even better still from 80% to 95% by weight, relative to the total weight of the composition. This amount depends on the formulation form of the desired composition. The amount of water may represent all or part of the aqueous phase, and it is generally at least 20% by weight relative to the total weight of the composition.

The aqueous phase may comprise at least one hydrophilic solvent, for instance substantially linear or branched lower monoalcohols containing from 1 to 8 carbon atoms, for instance ethanol, propanol, butanol, isopropanol or isobutanol; polyols such as propylene glycol, isoprene glycol, butylene glycol, glycerol, sorbitol or polyethylene glycols and derivatives thereof, and mixtures thereof.

In a known manner, all the compositions of the invention may contain one or more adjuvants that are customary in the cosmetic and dermatological fields, moisturizers; emollients; hydrophilic or lipophilic active agents; free-radical scavengers; sequestrants; antioxidants; preservatives; basifying or acidifying agents; fragrances; film-forming agents; colorants such as nacres and water-soluble dyes, and fillers; and mixtures thereof.

The amounts of these various adjuvants are those conventionally used in the fields under consideration. In particular, the amounts of active agents vary according to the desired objective and are those conventionally used in the fields under consideration, and for example from 0.1% to 20%, and preferably from 0.5% to 10% of the total weight of the composition.

Needless to say, a person skilled in the art will take care to select the optional adjuvant(s) added to the composition according to the invention such that the advantageous properties intrinsically associated with the composition in accordance with the invention are not, or are not substantially, adversely affected by the envisaged addition.

The composition preferably has a skin-friendly pH which generally ranges from 3 to 8 and preferably from 3.5 to 7.5.

The examples that follow will allow the invention to be understood more clearly, without, however, being limiting in nature. The raw materials are referred to by their chemical or INCI name. The amounts indicated are given as % by weight of raw materials, unless otherwise mentioned.

### Examples

### Example 1 (comparative): opaque emulsified gel

The following composition is prepared:

| | | |
|---|---|---|
| A | Demineralized water | QS |
| | Active agent(s) | 0.1 |
| | Dye(s) | 0.0004 |
| | Preservative(s) | 0.6 |
| | Sequestrant(s) | 0.25 |
| B | Glycerin | 5 |
| | Xanthan gum | 0.1 |
| | Carbopol (Synthalen K from 3V) | 0.5 |
| | Biosaccharide gum-1 (Fucogel 1.5P from SOLABIA) | 0.8 |
| C | Sodium hydroxide | 0.1 |
| D | Isononyl isononanoate | 1 |
| | Dimethicone | 3 |
| | Denatured Alcohol | 5 |
| E | Fragrance | 0.1 |
| | TOTAL | 100 |

### Procedure

This composition is manufactured with a customary procedure for emulsified gels.

In the main vessel, at low temperature, prepare phase A.

At low temperature, mix the ingredients of phase B and pour them into phase A using a turbomixer of Moritz type. Add sodium hydroxide in order to make the Carpobol gel swell.

At low temperature, additionally, mix the ingredients of phase D.

Pour the mixture thus obtained into the main vessel, with stirring using a turbomixer of Moritz type.

Finish by adding the fragrance.

The composition is in the form of a product of relatively thick consistency for presentation in a jar. The gel is smooth, shiny and opaque.

### Example 2 (invention): Translucent emulsified gel

The following composition is prepared:

| | | |
|---|---|---|
| A | Demineralized water | QS |
| | Active agent(s) | 0.1 |
| | Dye(s) | 0.0004 |
| | Preservative(s) | 0.6 |
| | Sequestrant(s) | 0.25 |
| B | Glycerin | 5 |
| | Xanthan gum | 0.1 |
| | Carbopol (Synthalen K from 3V) | 0.5 |
| | Biosaccharide gum-1 (Fucogel 1.5P from SOLABIA) | 0.8 |
| C | Sodium hydroxide | 0.1 |
| D | Isononyl isononanoate | 1 |
| | Dimethicone | 3 |
| | **Silica Silylate (Aerogel VM-2270 from Dow Corning)** | **0.3** |
| | Denatured Alcohol | 5 |
| E | Fragrance | 0.1 |
| | TOTAL | 100 |

### Procedure

This composition is manufactured with a customary procedure for emulsified gels.

In the main vessel, at low temperature, prepare phase A.

At low temperature, mix the ingredients of phase B and pour them into phase A using a turbomixer of Moritz type. Add sodium hydroxide in order to make the Carpobol gel swell.

At low temperature, additionally, mix the ingredients of phase D.

Pour the mixture thus obtained into the main vessel, with stirring using a turbomixer of Moritz type.

Finish by adding the fragrance.

The composition is in the form of a product of relatively thick consistency for presentation in a jar. The gel is smooth, shiny and translucent.

### CHARACTERIZATION OF THE TRANSLUCENCY AND STUDY OF THE PARAMETERS

In order to characterize the translucency, transmittance measurements are carried out in the visible range, using a spectrophotometer.

The measurements are carried out using a CARY 4000 (VARIAN) spectrophotometer over a wavelength range covering the visible range, between 400 and 800 nm. The samples are placed in PMMA cuvettes having an optical path length of 10 mm.

The results presented here are an average of the transmittances measured for each sample, over the whole of the wavelength range.

The transparency of the sample is considered to be satisfactory when the transmittance of the sample exceeds the value of 25% (¼ of the incident light at least is transmitted through the sample).

### A) Study of the influence of the ratio of the silica silylate content to the oil content (SS/O)

The amount and nature of the oils are constant.

The nature and the content of hydrophilic gelling agent remain identical for all the tests.

The ratio of the silica silylate content to the oil content is varied by modifying only the silica silylate content.

| | **Composition** | **A** | **B** | **C** |
|---|---|---|---|---|
| A | Demineralized water | QS | QS | QS |
| | Active agent(s) | 0.1 | 0.1 | 0.1 |
| | Dye(s) | 0.0004 | 0.0004 | 0.0004 |
| | Preservative(s) | 0.6 | 0.6 | 0.6 |
| | Sequestrant(s) | 0.25 | 0.25 | 0.25 |
| B | Glycerin | 5 | 5 | 5 |
| | Xanthan gum | 0.1 | 0.1 | 0.1 |
| | Carbopol (Synthalen K from 3V) | 0.5 | 0.5 | 0.5 |
| | Biosaccharide gum-1 (Fucogel 1.5P from SOLABIA) | 0.8 | 0.8 | 0.8 |
| C | Sodium hydroxide | 0.1 | 0.1 | 0.1 |
| D | Isononyl isononanoate | 1.25 | 1.25 | 1.25 |
| | Dimethicone | 3.75 | 3.75 | 3.75 |
| | **Silica Silylate (Aerogel VM-2270 from Dow Corning)** | **0.1** | **0.2** | **0.4** |
| | Denatured Alcohol | 5 | 5 | 5 |
| E | Fragrance | 0.1 | 0.1 | 0.1 |
| | TOTAL | 100 | 100 | 100 |
| | **SS/O RATIO** | **0.02** | **0.04** | **0.08** |
| | **Transmittance (%)** | **12.32** | **18.70** | **30.83** |

Compositions A and B are not translucent enough (transmittance values of less than 25%). Composition C according to the invention is translucent (transmittance value of greater than 25%).

The change in the transmittance as a function of the SS/O ratio is virtually linear. The minimum transmittance of 25% is achieved starting from an SS/O ratio of 0.06.

### B) Study of the influence of the nature of the thickening polymer

The contents of thickening polymers chosen are those customarily used for obtaining cosmetic gels.

The amount and nature of the oils are constant.

The ratio of the silica silylate content to the oil content remains constant at 0.075.

| | **Composition** | **D** | **E** | **F** | **G** | **H** | **I** |
|---|---|---|---|---|---|---|---|
| A | Demineralized water | QS | QS | QS | QS | QS | QS |
| | Active agent(s) | 0.1 | 0.1 | 0.1 | 0.1 | 0.1 | 0.1 |
| | Dye(s) | 0.0004 | 0.0004 | 0.0004 | 0.0004 | 0.0004 | 0.0004 |
| | Preservative(s) | 0.6 | 0.6 | 0.6 | 0.6 | 0.6 | 0.6 |
| | Sequestrant(s) | 0.25 | 0.25 | 0.25 | 0.25 | 0.25 | 0.25 |
| B | Glycerin | 5 | 5 | 5 | 5 | 5 | 5 |
| | Xanthan gum | 0.1 | 0.1 | 0.1 | 0.1 | 0.1 | 0.1 |
| | Biosaccharide gum-1 (Fucogel 1.5P from SOLABIA) | 0.8 | 0.8 | 0.8 | 0.8 | 0.8 | 0.8 |
| | **AMMONIUM POLYACRYLOYL DIMETHYL TAURATE (Hostacerin AMPS from Clariant)** | **0.8** | - | - | - | - | - |
| | **ACRYLATES/C10-30 ALKYL ACRYLATE CROSSPOLYMER (CARBOPOL ULTREZ 21 from LUBRIZOL)** | - | **0.5** | - | - | - | - |
| | **ACRYLATES/C10-30 ALKYL ACRYLATE CROSSPOLYMER (PEMULEN TR-1 from LUBRIZOL)** | - | - | **1.2** | - | - | - |
| | **AMMONIUM ACRYLOYLDIMETHYL TAURATE/STEARETH-8 METHACRYLATE COPOLYMER (Aristoflex SNC from CLARIANT)** | - | - | - | **1.0** | - | - |
| | **CARBOMER (Carbopol 981 from LUBRIZOL)** | - | - | - | - | **0.5** | - |
| | **Hydroxypropyl guar** | - | - | - | - | - | **1.0** |
| C | pH adjuster | qs pH 7.1 | qs pH 5.9 | qs pH 5.5 | qs pH 7.0 | qs pH 5.7 | qs pH 7.7 |
| D | Isononyl isononanoate | 1.0 | 1.0 | 1.0 | 1.0 | 1.0 | 1.0 |
| | Dimethicone | 3.0 | 3.0 | 3.0 | 3.0 | 3.0 | 3.0 |
| | Silica Silylate (Aerogel VM-2270 from Dow Corning) | 0.3 | 0.3 | 0.3 | 0.3 | 0.3 | 0.3 |
| | Denatured Alcohol | 5 | 5 | 5 | 5 | 5 | 5 |
| E | Fragrance | 0.1 | 0.1 | 0.1 | 0.1 | 0.1 | 0.1 |
| | TOTAL | 100 | 100 | 100 | 100 | 100 | 100 |
| | **Transmittance (%)** | **34.92** | **15.79** | **14.06** | **23.39** | **41.65** | **27.72** |

The thickening polymers having the most reduced emulsifying powers (Carbopol 981, Hostacerin AMPS, hydroxypropyl guar) provide the most translucent gels.

The thickening polymers with the highest emulsifying powers (Pemulen TR1, Aristoflex SNC, Carbopol Ultrez 21) give the least translucent gels.

### C) Study of the influence of the oil content

The nature of the oils is constant.

The gelling agent used is fixed.

The ratio of the silica silylate content to the oil content remains constant at 0.075.

| | **Composition** | **A** | **B** | **J** | **K** |
|---|---|---|---|---|---|
| A | Demineralized water | QS | QS | QS | QS |
| | Active agent(s) | 0.1 | 0.1 | 0.1 | 0.1 |
| | Dye(s) | 0.0004 | 0.0004 | 0.0004 | 0.0004 |
| | Preservative(s) | 0.6 | 0.6 | 0.6 | 0.6 |
| | Sequestrant(s) | 0.25 | 0.25 | 0.25 | 0.25 |
| B | Glycerin | 5 | 5 | 5 | 5 |
| | Xanthan gum | 0.1 | 0.1 | 0.1 | 0.1 |
| | Carbopol (Synthalen K from 3V) | 0.5 | 0.5 | 0.5 | 0.5 |
| | Biosaccharide gum-1 (Fucogel 1.5P from SOLABIA) | 0.8 | 0.8 | 0.8 | 0.8 |
| C | Sodium hydroxide | 0.1 | 0.1 | 0.1 | 0.1 |
| D | **Isononyl isononanoate** | **1** | **1** | **2.5** | **5.0** |
| | **Dimethicone** | **3** | **3** | **7.5** | **15.0** |
| | **Silica Silylate (Aerogel VM-2270 from Dow Corning)** | **-** | **0.3** | **0.75** | **1.5** |
| | Alcohol | 5 | 5 | 5 | 5 |
| E | Fragrance | 0.1 | 0.1 | 0.1 | 0.1 |
| | TOTAL | 100 | 100 | 100 | 100 |
| | **Oil content** | **4** | **4** | **10** | **20** |
| | **Transmittance (%)** | **11.30** | **27.03** | **33.08** | **39.26** |

By keeping the SS/O ratio equal to 0.075, it is possible to increase the oil content up to 20% without loss of transparency.

### Conclusion

By introducing a small amount of silica silylate into a traditionally opaque emulsified gel, it is possible to obtain compositions that are much more translucent while retaining the remainder of the organoleptic properties of the product.

## Claims

1. A translucent cosmetic composition in the form of an oil-in-water emulsion comprising:
(1) a dispersed fatty phase comprising at least one oil selected from silicone oils and fatty acid esters;
(2) a continuous aqueous phase comprising at least one hydrophilic thickening polymer; and
(3) hydrophobic silica aerogel particles;
the ratio of the amount by weight of silica aerogel particles to the amount by weight of oil(s) defined in (1) being greater than 0.06;
the composition being free of surfactant(s).

2. The composition as claimed in claim 1, wherein the hydrophilic thickening polymer(s) is (are) not amphiphilic.

3. The composition as claimed in either one of claims 1 and 2, wherein the hydrophilic thickening polymer(s) is (are) selected from:
1) acrylic acid homopolymers or copolymers, which are preferably crosslinked, and salts thereof;
2) polyacrylate salts;
3) 2-acrylamido-2-methylpropanesulfonic acid (AMPS®) polymers and copolymers, which are optionally crosslinked and/or neutralized, selected from:
a) poly(2-acrylamido-2-methylpropanesulfonic acid) polymers, in particular homopolymers, polyAMPS®, which are crosslinked and neutralized to at least 90%;
b) copolymers of acrylamide and of AMPS®, in particular crosslinked anionic copolymers of acrylamide and of AMPS®;
4) polysaccharides, for example heterogeneous polysaccharides, and polysaccharides such as gums, in particular xanthan gum or hydroxypropyl guar;
5) glyceryl acrylate polymers.

4. The composition as claimed in claim 3, wherein the hydrophilic thickening polymer(s) is (are) selected from:
1) acrylic acid homopolymers or copolymers, which are preferably crosslinked, and salts thereof;
2) polyacrylate salts;
3) 2-acrylamido-2-methylpropanesulfonic acid (AMPS®) polymers and copolymers, which are optionally crosslinked and/or neutralized, selected from:
a) poly(2-acrylamido-2-methylpropanesulfonic acid) polymers, in particular homopolymers, polyAMPS®, which are crosslinked and neutralized to at least 90%;
b) copolymers of acrylamide and of AMPS®, in particular crosslinked anionic copolymers of acrylamide and of AMPS®;
4) polysaccharides such as gums, in particular hydroxypropyl guar.

5. The composition as claimed in claim 4, wherein the hydrophilic thickening polymer(s) is (are) selected from: acrylic acid homopolymers or copolymers, which are preferably crosslinked, and salts thereof; 2-acrylamido-2-methylpropanesulfonic acid (AMPS®) polymers and copolymers, which are optionally crosslinked and/or neutralized, selected from poly(2-acrylamido-2-methylpropanesulfonic acid) polymers, in particular homopolymers, polyAMPS®, which are crosslinked and neutralized to at least 90%, and copolymers of acrylamide and of AMPS®, in particular crosslinked anionic copolymers of acrylamide and of AMPS®.

6. The composition as claimed in any one of claims 1 to 5, wherein the hydrophobic silica aerogel particles have a specific surface area per unit of mass S_{M} ranging from 200 to 1500 m²/g, preferably from 600 to 1200 m²/g and better still from 600 to 800 m²/g.

7. The composition as claimed in any one of claims 1 to 6, wherein the hydrophobic silica aerogel particles have a size, expressed as the volume-average diameter (D[0.5]), of less than 1500 µm, preferably ranging from 5 to 25 µm and better still from 5 to 20 µm.

8. The composition as claimed in any one of claims 1 to 7, wherein the hydrophobic silica aerogel particles have a tapped density ranging from 0.02 g/cm³ to 0.10 g/cm³ and preferably from 0.03 g/cm³ to 0.08 g/cm³.

9. The composition as claimed in any one of claims 1 to 8, wherein the hydrophobic silica aerogel particles have a specific surface area per unit of volume S_{V} ranging from 5 to 60 m²/cm³, preferably from 10 to 50 m²/cm³ and better still from 15 to 40 m²/cm³.

10. The composition as claimed in any one of claims 1 to 9, wherein the hydrophobic silica aerogel particles have an oil absorption capacity, measured at the wet point, ranging from 5 to 18 ml/g, preferably from 6 to 15 ml/g and better still from 8 to 12 ml/g of particles.

11. The composition as claimed in any one of claims 1 to 10, wherein the hydrophobic silica aerogel particles are surface-modified with trimethylsilyl groups.

12. The composition as claimed in any one of claims 1 to 11, wherein the hydrophobic silica aerogel particles are present in a content ranging from 0.001% to 10% by weight, preferably from 0.005% to 5% by weight, and better still from 0.1% to 2% by weight relative to the total weight of the composition.

13. The composition as claimed in any one of claims 1 to 12, wherein the proportion of the fatty phase is less than or equal to 25% by weight, preferably from 0.1% to 20% by weight and better still from 1% to 20% by weight relative to the total weight of the composition.

14. A method for the cosmetic treatment of a keratin material, wherein a composition as defined in any one of claims 1 to 13 is applied to the keratin material.

## Patentansprüche

1. Transluzente kosmetische Zusammensetzung in Form einer Öl-in-Wasser-Emulsion, umfassend:
(1) eine disperse Fettphase, umfassend mindestens ein Öl, das aus Silikonölen und Fettsäureestern ausgewählt ist;
(2) eine kontinuierliche wässrige Phase, umfassend mindestens ein hydrophiles verdickendes Polymer; und
(3) Aerogelteilchen aus hydrophobem Siliciumdioxid;
wobei das Verhältnis der Gewichtsmenge von Siliciumdioxid-Aerogelteilchen zur Gewichtsmenge von Öl(en) gemäß (1) größer als 0,06 ist;
wobei die Zusammensetzung frei von Tensid(en) ist.

2. Zusammensetzung nach Anspruch 1, wobei das hydrophile verdickende Polymer bzw. die hydrophilen verdickenden Polymere nicht amphiphil ist bzw. sind.

3. Zusammensetzung nach einem der Ansprüche 1 und 2, wobei das hydrophile verdickende Polymer bzw. die hydrophilen verdickenden Polymere aus
1) Acrylsäure-Homopolymeren oder -Copolymeren, die vorzugsweise vernetzt sind, und Salzen davon,
2) Polyacrylatsalzen;
3) 2-Acrylamido-2-methylpropansulfonsäure(AMPS®) - Polymeren und -Copolymeren, die gegebenenfalls vernetzt und/oder neutralisiert sind, ausgewählt aus
a) Poly(2-acrylamido-2-methylpropansulfonsäure)-Polymeren, insbesondere Homopolymeren, PolyAMPS®, die vernetzt und zu mindestens 90 % neutralisiert sind;
b) Copolymeren von Acrylamid und AMPS®, insbesondere vernetzten anionischen Copolymeren von Acrylamid und AMPS®;
4) Polysacchariden, beispielsweise heterogenen Polysacchariden, und Polysacchariden wie Gummen, insbesondere Xanthangummi oder Hydroxypropylguar;
5) Glycerylacrylat-Polymeren
ausgewählt ist bzw. sind.

4. Zusammensetzung nach Anspruch 3, wobei das hydrophile verdickende Polymer bzw. die hydrophilen verdickenden Polymere aus
1) Acrylsäure-Homopolymeren oder -Copolymeren, die vorzugsweise vernetzt sind, und Salzen davon,
2) Polyacrylatsalzen;
3) 2-Acrylamido-2-methylpropansulfonsäure(AMPS®) - Polymeren und -Copolymeren, die gegebenenfalls vernetzt und/oder neutralisiert sind, ausgewählt aus
a) Poly(2-acrylamido-2-methylpropansulfonsäure)-Polymeren, insbesondere Homopolymeren, PolyAMPS®, die vernetzt und zu mindestens 90 % neutralisiert sind;
b) Copolymeren von Acrylamid und AMPS®, insbesondere vernetzten anionischen Copolymeren von Acrylamid und AMPS®;
4) Polysacchariden wie Gummen, insbesondere Hydroxypropylguar
ausgewählt ist bzw. sind.

5. Zusammensetzung nach Anspruch 4, wobei das hydrophile verdickende Polymer bzw. die hydrophilen verdickenden Polymere aus Acrylsäure-Homopolymeren und -Copolymeren, die vorzugsweise vernetzt sind, und Salzen davon, 2-Acrylamido-2-methylpropansulfonsäure (AMPS®) -Polymeren und -Copolymeren, die gegebenenfalls vernetzt und/oder neutralisiert sind, ausgewählt aus Poly(2-acrylamido-2-methylpropansulfonsäure)-Polymeren, insbesondere Homopolymeren, PolyAMPS®, die vernetzt und zu mindestens 90 % neutralisiert sind, und Copolymeren von Acrylamid und AMPS®, insbesondere vernetzten anionischen Copolymeren von Acrylamid und AMPS® ausgewählt ist bzw. sind.

6. Zusammensetzung nach einem der Ansprüche 1 bis 5, wobei die Aerogelteilchen aus hydrophobem Siliciumdioxid eine spezifische Oberfläche pro Masseneinheit S_{M} im Bereich von 200 bis 1500 m²/g, vorzugsweise von 600 bis 1200 m²/g und noch besser von 600 bis 800 m²/g aufweisen.

7. Zusammensetzung nach einem der Ansprüche 1 bis 6, wobei die Aerogelteilchen aus hydrophobem Siliciumdioxid eine als volumenmittlerer Durchmesser (D[0,5]) ausgedrückte Größe von weniger als 1500 µm und vorzugsweise im Bereich von 5 bis 25 µm und noch besser von 5 bis 20 µm aufweisen.

8. Zusammensetzung nach einem der Ansprüche 1 bis 7, wobei die Aerogelteilchen aus hydrophobem Siliciumdioxid eine Klopfdichte im Bereich von 0,02 g/cm³ bis 0,10 g/cm³ und vorzugsweise von 0,03 g/cm³ bis 0,08 g/cm³ aufweisen.

9. Zusammensetzung nach einem der Ansprüche 1 bis 8, wobei die Aerogelteilchen aus hydrophobem Siliciumdioxid eine spezifische Oberfläche pro Volumeneinheit S_{V} im Bereich von 5 bis 60 m²/cm³, vorzugsweise von 10 bis 50 m²/cm³ und noch besser von 15 bis 40 m²/cm³ aufweisen.

10. Zusammensetzung nach einem der Ansprüche 1 bis 9, wobei die Aerogelteilchen aus hydrophobem Siliciumdioxid eine am Wet Point gemessene Ölabsorptionskapazität im Bereich von 5 bis 18 ml/g, vorzugsweise von 6 bis 15 ml/g und noch besser von 8 bis 12 ml/g Teilchen aufweisen.

11. Zusammensetzung nach einem der Ansprüche 1 bis 10, wobei die Aerogelteilchen aus hydrophobem Siliciumdioxid mit Trimethylsilylgruppen oberflächenmodifiziert sind.

12. Zusammensetzung nach einem der Ansprüche 1 bis 11, wobei die Aerogelteilchen aus hydrophobem Siliciumdioxid in einem Gehalt im Bereich von 0,001 bis 10 Gew.-%, vorzugsweise von 0,005 bis 5 Gew.-% und noch besser von 0,1 bis 2 Gew.-%, bezogen auf das Gesamtgewicht der Zusammensetzung, vorliegen.

13. Zusammensetzung nach einem der Ansprüche 1 bis 12, wobei der Anteil der Fettphase kleiner oder gleich 25 Gew.-% ist und vorzugsweise 0,1 bis 20 Gew.-% und noch besser 1 bis 20 Gew.-%, beträgt, bezogen auf das Gesamtgewicht der Zusammensetzung.

14. Verfahren zur kosmetischen Behandlung eines Keratinmaterials, bei dem man auf das Keratinmaterial eine Zusammensetzung gemäß einem der Ansprüche 1 bis 13 aufbringt.

## Revendications

1. Composition cosmétique translucide sous la forme d'une émulsion de type huile-dans-eau comprenant :
(1) une phase grasse dispersée comprenant au moins une huile choisie parmi des huiles de silicone et des esters d'acide gras ;
(2) une phase aqueuse continue comprenant au moins un polymère épaississant hydrophile ; et
(3) des particules d'aérogel de silice hydrophobe ;
le ratio de la quantité en poids de particules d'aérogel de silice sur la quantité en poids d'huile(s) définies en (1) étant supérieur à 0,06 ;
la composition étant exempte de tensio-actif(s).

2. Composition selon la revendication 1, dans laquelle le(s) polymère(s) épaississant(s) hydrophile(s) n'est/ne sont pas amphiphile(s).

3. Composition selon l'une ou l'autre des revendications 1 et 2 dans laquelle le(s) polymère(s) épaississant(s) hydrophile(s) est/sont choisi(s) parmi :
1) des homopolymères ou copolymères d'acide acrylique, qui sont préférablement réticulés, et leurs sels ;
2) des sels de polyacrylate ;
3) des polymères et copolymères d'acide 2-acrylamido 2-méthylpropanesulfonique (AMPS®), qui sont éventuellement réticulés et/ou neutralisés, choisis parmi :
a) des polymères de type poly(acide 2-acrylamido-2-méthylpropanesulfonique), en particulier des homopolymères, polyAMPS®, réticulés et neutralisés à raison d'au moins 90 % ;
b) des copolymères d'acrylamide et d'AMPS®, en particulier des copolymères anioniques réticulés d'acrylamide et d'AMPS® ;
4) des polysaccharides, par exemple des polysaccharides hétérogènes, et des polysaccharides tels que des gommes, en particulier la gomme de xanthane ou l'hydroxypropylguar ;
5) des polymères d'acrylate de glycéryle.

4. Composition selon la revendication 3, dans laquelle le(s) polymère(s) épaississant(s) hydrophile(s) est/sont choisi(s) parmi :
1) des homopolymères ou copolymères d'acide acrylique, qui sont préférablement réticulés, et leurs sels ;
2) des sels de polyacrylate ;
3) des polymères et copolymères d'acide 2-acrylamido-2-méthylpropanesulfonique (AMPS®), qui sont éventuellement réticulés et/ou neutralisés, choisis parmi :
a) des polymères de type poly(acide 2-acrylamido-2-méthylpropanesulfonique), en particulier des homopolymères, polyAMPS®, réticulés et neutralisés à raison d'au moins 90 % ;
b) des copolymères d'acrylamide et d'AMPS®, en particulier des copolymères anioniques réticulés d'acrylamide et d'AMPS® ;
4) des polysaccharides tels que des gommes, en particulier l'hydroxypropylguar.

5. Composition selon la revendication 4, dans laquelle le(s) polymère(s) épaississant(s) hydrophile(s) est (sont) choisi(s) parmi : des homopolymères ou copolymères d'acide acrylique, qui sont préférablement réticulés, et leurs sels; des polymères et copolymères d'acide 2-acrylamido-2-méthylpropanesulfonique (AMPS®), qui sont éventuellement réticulés et/ou neutralisés, choisis parmi des polymères de type poly(acide 2-acrylamido-2-méthylpropanesulfonique), en particulier des homopolymères, polyAMPS®, réticulés et neutralisés à raison d'au moins 90 %, et des copolymères d'acrylamide et d'AMPS®, en particulier des copolymères anioniques réticulés d'acrylamide et d'AMPS®.

6. Composition selon l'une quelconque des revendications 1 à 5, dans laquelle les particules d'aérogel de silice hydrophobe possèdent une surface spécifique par unité de masse S_{M} dans la plage de 200 à 1 500 m²/g, préférablement de 600 à 1 200 m²/g et encore mieux de 600 à 800 m²/g.

7. Composition selon l'une quelconque des revendications 1 à 6, dans laquelle les particules d'aérogel de silice hydrophobe possèdent une grosseur, exprimée en diamètre moyen en volume (D[0,5]), inférieure à 1 500 µm, préférablement dans la plage de 5 à 25 µm et encore mieux de 5 à 20 µm.

8. Composition selon l'une quelconque des revendications 1 à 7, dans laquelle les particules d'aérogel de silice hydrophobe possèdent une densité tassée dans la plage de 0,02 g/cm³ à 0,10 g/cm³, et préférablement de 0,03 g/cm³ à 0,08 g/cm³.

9. Composition selon l'une quelconque des revendications 1 à 8, dans laquelle les particules d'aérogel de silice hydrophobe possèdent une surface spécifique par unité de volume S_{V} dans la plage de 5 à 60 m²/cm³, préférablement de 10 à 50 m²/cm³ et encore mieux de 15 à 40 m²/cm³.

10. Composition selon l'une quelconque des revendications 1 à 9, dans laquelle les particules d'aérogel de silice hydrophobe possèdent une capacité d'absorption d'huile, mesurée au point de mouillage dans la plage de 5 à 18 ml/g, préférablement de 6 à 15 ml/g et encore mieux de 8 à 12 ml/g, de particules.

11. Composition selon l'une quelconque des revendications 1 à 10, dans laquelle les particules d'aérogel de silice hydrophobe sont modifiées en surface par des groupes triméthylsilyle.

12. Composition selon l'une quelconque des revendications 1 à 11, dans laquelle les particules d'aérogels de silice hydrophobe sont présentes en une teneur dans la plage de 0,001 % à 10 % en poids, préférablement de 0,005 % à 5 % en poids, et encore mieux de 0,1 % à 2 % en poids par rapport au poids total de la composition.

13. Composition selon l'une quelconque des revendications 1 à 12 dans laquelle la proportion de la phase grasse est inférieure ou égale à 25 % en poids, préférablement de 0,1 % à 20 % en poids et encore mieux de 1 % à 20 % en poids par rapport au poids total de la composition.

14. Procédé pour le traitement cosmétique d'une matière kératinique, dans lequel une composition telle que définie dans l'une quelconque des revendications 1 à 13 est appliquée à la matière kératinique.
